# EUROPEAN PATENT SPECIFICATION

(11) **EP 0 822 181 B1**
(45) Date of publication and mention of the grant of the patent: **02.05.2001**
(21) Application number: 96202170.5
(22) Date of filing: 01.08.1996
(51) Int. Cl.: C07C 273/04, B01J 12/02

(54) **Process and plant for the production of urea with high conversion yield and low energy consumption**
Verfahren und Anlage zur Herstellung von Harnstoff mit hohem Umwandlungsgrad und niedrigem Energieverbrauch
Procédé et installation pour la fabrication d'urée à rendement de conversion élevé et faible consommation d'énergie

(43) Date of publication of application: 04.02.1998
(73) Proprietor: UREA CASALE S.A., 6900 Lugano-Besso (CH)
(72) Inventor: Pagani, Giorgio, 20142 Milano (IT)
(74) Representative: Zardi, Marco

(56) References cited:
- EP-A- 0 479 103
- EP-A- 0 497 215
- EP-A- 0 611 753
- EP-A- 0 624 571
- NL-A- 6 808 167

## Description

In its general aspect the present invention relates to a process for urea production.

The present invention relates specifically to a process for urea production of the type comprising the steps of:
- performing a reaction between ammonia and carbon dioxide in a first reaction space to obtain a reaction mixture comprising urea, carbamate and free ammonia in aqueous solution;
- feeding said mixture to a urea recovery section;
- separating in said recovery section said carbamate and free ammonia in aqueous solution from the urea.

The present invention also relates to a plant for carrying out the above mentioned process and to a method for modernizing an existing urea plant to obtain a plant according to the present invention.

As known, in the field of urea production the need is ever more growing of plants having greater capacity and operating flexibility on the one hand, on the other hand, requiring ever smaller investment and operating costs, in particular in energy terms.

### Prior Art

To this end, there have been proposed and implemented in the art a series of urea production processes essentially based on the performance of a conversion reaction in a reaction space fed with ammonia (NH₃) and carbon dioxide (CO₂) and to which are recycled the unreacted substances contained in the urea solution leaving the reaction space, in particular ammonia, carbon dioxide and dilute carbamate in aqueous solution.

A process of this type comprises downstream to a reaction space, a urea recovery section for separating from the urea solution the unreacted substances to be recycled.

As an alternative, there have been proposed processes for the urea production essentially based on the performance of conversion reactions with differentiated yields in two reaction spaces placed in parallel with each other, as described for example in European Patent Application EP-A-0 479 103.

In this case, the unreacted substances are all recycled to one of the reactor spaces.

If, on the one hand, this recycle allows almost complete recovery of valuable substances such as ammonia and carbon dioxide, on the other hand it also involves the sending to the reactor of large quantities of water (H20) which are detrimental to the overall yield of conversion of the carbon dioxide to urea, with the yield being generally between 55% and 62%.

### Summary of the invention

The technical problem underlying the present invention is accordingly to conceive and make available a process for urea production achieving high conversion yield which would be technically simple to implement, and would involve low energy consumption and operating costs.

In accordance with the present invention, this problem is solved by a process of the above mentioned type which is characterized in that it comprises the additional steps of:
- subjecting at least part of said carbamate and free ammonia in aqueous solution obtained in the urea recovery section to a treatment of partial decomposition of the carbamate and partial separation of the free ammonia to obtain a flow comprising ammonia and carbon dioxide in vapor phase and a flow comprising residual carbamate in aqueous solution;
- subjecting said flow comprising ammonia and carbon dioxide in vapor phase to at least partial condensation to obtain a flow of concentrate carbamate in aqueous solution;
- feeding at least part of said concentrate carbamate to a second reaction space;
- performing a reaction between ammonia and carbon dioxide in said second reaction space to obtain a reaction mixture comprising urea, carbamate and free ammonia in aqueous solution.

In the following description and in the subsequent claims, with the term: "concentrate" carbamate in aqueous solution it is intended to mean a carbamate solution with a very low content of water (a few percentage points).

According to this invention, at least part of the carbamate and free ammonia in aqueous solution leaving the urea recovery section is advantageously subjected to a treatment of partial decomposition separating unreacted ammonia and carbon dioxide from a solution rich in water comprising residual carbamate.

So doing, the unreacted substances to be recycled have a very low water content, and thus it is possible to substantially limit the water fed to the reaction space, permitting a high conversion yield.

Advantageously, these unreacted substances are recycled to a second reaction space wherein a reaction between ammonia and carbon dioxide is performed.

Thanks to the presence of first reaction space fed with substantially pure reagents and of a second reaction space which is fed with concentrate carbamate having a very low water content, it is possible to obtain a further increase in the overall conversion yield.

In order to obtain a high degree of decomposition of the carbamate and separation of the free ammonia in aqueous solution, the treatment of partial decomposition and separation is preferably carried out at a pressure substantially corresponding to the pressure in the second reaction space.

To improve and assist the condensation and separation steps of the unreacted substances in the urea recovery section, the flow comprising residual carbamate in aqueous solution resulting from the treatment of partial decomposition of the carbamate and partial separation of the free ammonia is advantageously fed to said urea recovery section.

According to another aspect of the present invention, the technical problem set forth above is solved by a plant designed to implement the above mentioned urea production process comprising:
- a first urea synthesis reactor;
- a second urea synthesis reactor;
- a recovery section of a reaction mixture comprising urea, carbamate and free ammonia in aqueous solution coming from said first urea synthesis reactor, for separating the urea from said carbamate and free ammonia in aqueous solution;
which plant is characterized in that it comprises:
- a stripping unit for subjecting at least part of said carbamate and free ammonia in aqueous solution obtained in the urea recovery section to a treatment of partial decomposition of the carbamate and partial separation of the free ammonia to obtain a flow comprising ammonia and carbon dioxide in vapor phase and a flow comprising residual carbamate in aqueous solution;
- means for condensing at least partially the vapors leaving said stripping unit to obtain a flow of concentrate carbamate in aqueous solution;
- means for feeding at least part of said concentrate carbamate to the second urea synthesis reactor.

In accordance with the present invention the plants delegated to carry out the urea production process may be provided either new or by modifying pre-existing plants so as to obtain a production capacity expansion and at the same time improved performance from the energy consumption viewpoint.

Accordingly, the present invention makes available a method for modernizing a urea production plant of the type comprising:
- a first urea synthesis reactor;
- a recovery section of a reaction mixture comprising urea, carbamate and free ammonia in aqueous solution coming from said first urea synthesis reactor, for separating the urea from said carbamate and free ammonia in aqueous solution;
which method is characterized in that it comprises the steps of:
- providing a second urea synthesis reactor;
- providing a stripping unit for subjecting at least part of said carbamate and free ammonia in aqueous solution obtained in the urea recovery section to a treatment of partial decomposition of the carbamate and partial separation of the free ammonia to obtain a flow comprising ammonia and carbon dioxide in vapor phase and a flow comprising residual carbamate in aqueous solution;
- providing means for condensing at least partially the vapors leaving said stripping unit to obtain a flow of concentrate carbamate in aqueous solution;
- providing means for feeding at least part of said concentrate carbamate to the second urea synthesis reactor.

Further characteristics and advantages of the present invention are set forth in the detailed description of an embodiment thereof given below by way of non-limiting example with reference to the annexed figure.

### Brief description of the figure

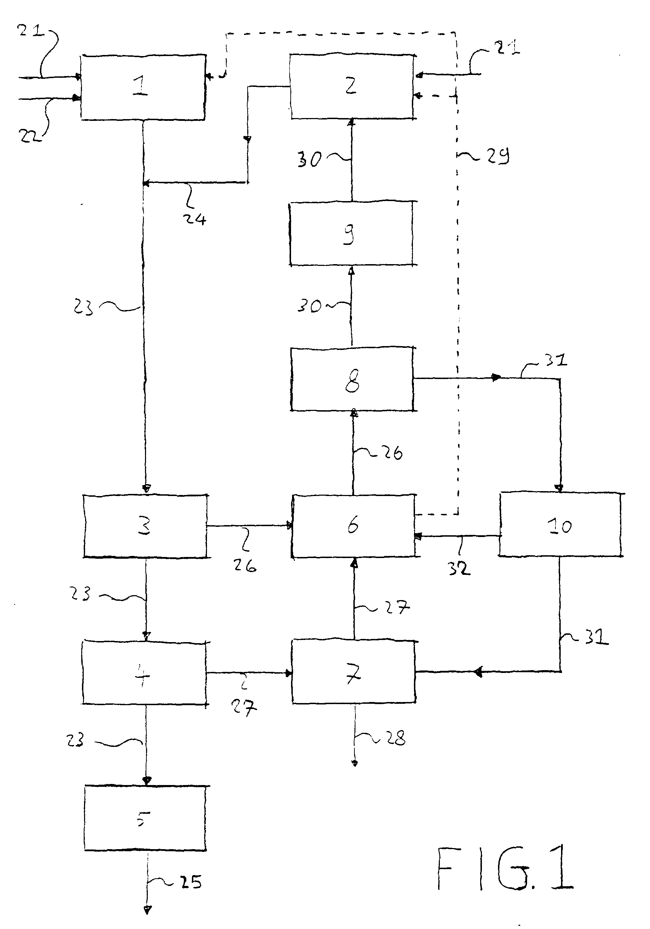

FIG. 1 shows a block diagram of a urea production process according to present invention.

### Detailed description of a preferred embodiment

In FIG. 1 blocks 1 and 2 indicate high pressure reaction spaces for the synthesis of urea.

Typical operating conditions in the reaction space 1 are:
- molar ratio NH₃/CO₂ at input: 3.6;
- molar ratio H₂O/CO₂ at input: 0;
- conversion yield of the CO₂ into urea: 75%;
- pressure: 240 bar a;
- temperature: 195°C.

The reaction space 1 is advantageously of the once-through type and is fed by gas flows 21 and 22 comprising substantially pure ammonia and carbon dioxide respectively.

In the following description, with the term: "once-through" type reaction space it is intended to mean a reaction space substantially without recycle of the unreacted reagents, in particular carbamate in aqueous solution.

Preferably, the gas flows 21 and 22 traverse a pre-condenser (not shown) of the Kettle type before being fed to the reaction space 1, in order to remove the heat in excess and producing steam at 6-9 bar a.

Typical operating conditions in the reaction space 2 are:
- molar ratio NH₃/CO₂ at input: 3.2;
- molar ratio H₂O/CO₂ at input: 0.1;
- conversion yield of the CO₂ into urea: 72%;
- pressure: 150 bar a;
- temperature: 190°C.

Advantageously, the reaction space 2 is fed by gas flows 21 and 30 comprising substantially pure ammonia and concentrate carbamate in aqueous solution respectively.

As shown in the figure, reactions spaces 1 and 2 are operated in parallel.

In this example, about 75% of the overall reaction is performed in the first reaction space 1 and about 25% in the second reaction space 2.

Blocks 5 and 9 indicate respectively a urea granulation or prilling section and a high pressure condensation units.

The condensation unit 9 is preferably of the Kettle type in order to remove the heat in excess and producing steam at 5-6 bar a, and operates at the same pressure conditions as the reaction space 2.

A urea recovery section is generally indicated by blocks 3, 4, 6 and 7. In particular, blocks 3 and 4 indicate a distillation unit and blocks 6 and 7 indicate a condensation unit.

Block 4 also indicates a urea finishing unit, wherein a solution with an urea content of up to 99.7% is obtained.

Block 7 also indicates a waste water treatment unit for purification of the water to be discharged from the urea production process.

Typically, blocks 3 and 6 operate at medium pressure (about 18 bar), while blocks 4 and 7 operate at low pressure (about 3 bar).

In accordance with an alternative embodiment of the process, block 6 further comprises an ammonia separation column to obtain a substantially pure liquid ammonia which is sent to the reaction spaces 1 and 2 in addition to the flow 21, as indicated by flow line 29 in FIG. 1.

According to the present invention, block 8 indicates a high pressure decomposition unit operating advantageously at the same pressure conditions as the reaction space 2.

This decomposition unit 8 generally comprises a stripper apparatus.

Flow lines 23 and 24 represent a liquid flow of a reaction mixture coming from blocks 1 and 2 respectively, and comprising urea and unreacted substances, notably carbamate and free ammonia in aqueous solution.

Flow line 24 is poured into flow 23 which traverses the distillation units of the urea recovery section indicated by blocks 3 and 4, where the carbamate is decomposed and separated together with the free ammonia from the urea solution.

Generally, the urea content in the liquid flow 23 is between 70%-72% after block 3 and about 99% after block 4.

Flow lines 26 and 27 represent a gas flow comprising ammonia and carbon dioxide in gaseous phase obtained in blocks 3 and 4 respectively.

The flow 27 traverses the condensation unit represented by block 7, where the ammonia and carbon dioxide in vapor phase are condensed obtaining a flow of carbamate in aqueous solution, and is fed to the condensation unit 6, where it promotes condensation of the gas flow 26.

Analogously, the flow 26 traverses the condensation unit represented by block 6, where the ammonia and carbon dioxide in vapor phase are condensed obtaining a flow of carbamate and free ammonia in aqueous solution having a high water content, and is fed to the decomposition unit 8, where it is advantageously subjected to a treatment of partial decomposition of the carbamate and partial separation of the free ammonia.

Part of the water contained in the aqueous solution obtained in the condensation unit of block 7 is further treated and purified of almost all traces of ammonia and urea in the treatment unit also indicated by block 7. From block 7 departs a flow line 28 of a waste water flow to be discharged from the urea production process.

The urea solution flow 23 coming from block 4 traverses the granulation or prilling section indicated by block 5, where it is transformed to a final product leaving the urea production process by flow line 25.

At the outlet of block 8, flow lines 30 and 31 are shown which represent respectively a gas flow comprising ammonia and carbon dioxide in vapor phase and a liquid flow comprising urea and residual carbamate in aqueous solution.

The gas flow 30, which is very rich in ammonia and carbon dioxide and poor in water (only a few percentages points), traverses the condensation unit represented by block 6, where ammonia and carbon dioxide are condensed obtaining a flow of concentrate carbamate in aqueous solution, and is recycled to the reaction space 2 through flow line 30.

In the example of FIG. 1, all the carbamate in aqueous solution separated from the urea in the recovery section is subjected to the decomposition treatment in block 8. However, satisfactory results have been obtained also by feeding to block 8 only a part of carbamate leaving the urea recovery section. For instance, between 50% to 90% of this carbamate may be sent to block 8.

According to the process of the present invention, a reaction between ammonia and carbon dioxide is performed in a first reaction space 1 obtaining a reaction mixture comprising urea, carbamate and free ammonia in aqueous solution, which is fed to a urea recovery section (blocks 3, 4, 6, 7) where the urea is separated from the carbamate and the free ammonia in aqueous solution (flow line 26).

Advantageously, in accordance with other process steps of the present invention, at least part of the flow 26 is further subjected to a treatment of partial decomposition in block 8 to obtain a flow 30 comprising ammonia and carbon dioxide in vapor phase and a flow 31 comprising residual carbamate in aqueous solution. The flow 30 is then at least partially condensed in block 9 to obtain a flow of concentrate carbamate in aqueous solution which is recycled to a second reaction space 2 via flow line 30.

The flow 30 of concentrate carbamate fed to block 2, generally comprises free ammonia as well.

By operating in this manner it is possible to obtain a high conversion yield in the first and also in the second reaction space 1 respectively 2, since the unreacted reagents are advantageously recycled to the second reaction space substantially free of water.

According to the present urea production process, it is possible to achieve an overall conversion yield of carbon dioxide to urea of about 70% to 76%, which is notably greater than that obtainable with the prior art processes.

Moreover, this high conversion yield and the substantially absence of water to be recycled to the reaction space 2 also result in a smaller flow of substances to be separated from the urea solution, and thus it result in an increase in the performance of the distillation units 3 and 4 of the recovery section.

In accordance with the present invention, the liquid flow 31, which is very rich in water, is advantageously recycled to the urea recovery section in order to promote condensation and recover of the unreacted substances which are freed in the distillation units 3 and 4.

Preferably, flow line 31 traverses a distillation unit indicated by block 10, where the residual carbamate is further subjected to decomposition in order to obtain a solution very rich in water which is fed to block 7.

From block 10 also departs a flow line 32 of a water poor vapor flow comprising residual ammonia and carbon dioxide which is fed to the condensation unit indicated by block 6 of the urea recovery section.

There is thus obtained a separate circulation loop of process water which advantageously promotes condensation of ammonia and carbon dioxide vapors in condensation units 6 and 7 without being recycled to the reaction spaces 1 or 2 and thus without negatively affecting the reaction between ammonia and carbon dioxide.

An additional distillation unit - not shown - may be provided between blocks 10 and 7 in order to further decompose the flow 31 very rich in water leaving block 10.

In this case, a water poor vapor flow comprising residual ammonia and carbon dioxide is fed to the condensation unit indicated by block 7, while a solution very rich in water is fed to the waste water treatment unit also indicated by 7.

Preferably, the distillation unit 10 and the additional distillation unit operate at medium and low pressure respectively.

The urea recovery section may also comprise an additional low pressure (about 1-2 bar a) distillation and condensation unit - not shown - disposed downstream blocks 4 and 7 respectively.

Reference is now made to a plant for urea production specifically designed to carry out the process according to the present invention.

The urea production plant advantageously comprises two urea synthesis reactors indicated by blocks 1 and 2, a urea recovery section indicated by blocks 3, 4, 6 and 7, a stripping unit indicated by block 8 and respective means for condensing and recycling to the reactor 2 the vapors leaving the stripping unit (block 9, flow line 30).

With reference to the figure, the plant advantageously comprises means for feeding a flow comprising residual carbamate in aqueous solution from the stripping unit to the recovery section.

Preferably, a distiller unit indicated by block 10 is disposed between the stripping unit and the recovery section.

Moreover, the plant also comprises means (flow line 24) for feeding a reaction mixture comprising urea, carbamate and free ammonia in aqueous solution leaving the second reactor to the urea recovery section.

The plant designed to implement the process for urea production in accordance with the present invention may be a brand new plant or a plant obtained by modernizing a pre-existing plant.

Accordingly, this modernization takes place by means of the following steps:
- providing a second urea synthesis reactor (block 2);
- providing a stripping unit (block 8) for subjecting at least part of said carbamate and free ammonia in aqueous solution (flow line 26) obtained in the urea recovery section (blocks 3, 4, 6 and 7) to a treatment of partial decomposition of the carbamate and partial separation of the free ammonia to obtain a flow comprising ammonia and carbon dioxide in vapor phase and a flow comprising residual carbamate in aqueous solution (flow lines 30 and 31 respectively) ;
- providing means (block 9) for condensing at least partially the vapors leaving said stripping unit (block 8) to obtain a flow of concentrate carbamate in aqueous solution;
- providing means (flow line 30) for feeding at least part of said concentrate carbamate to the second urea synthesis reactor (block 2).

Advantageously, the method for modernizing a pre-existing plant additionally comprises the step of:
- providing means (flow line 31) for feeding said flow comprising residual carbamate in aqueous solution from said stripping unit (block 8) to said recovery section (blocks 3, 4, 6 and 7).

According to a further embodiment of the present invention, the modernizing method comprises the step of:
- providing means (flow line 24) for feeding a reaction mixture comprising urea, carbamate and free ammonia in aqueous solution leaving the second synthesis reactor (block 2) to said urea recovery section (blocks 3, 4, 6 and 7).

Thanks to the modernizing method of the present invention, not only the conversion yield of the pre-existing urea synthesis reactor can be drastically increased but also its capacity.

In fact, a bigger flow of reagents may be fed to the pre-existing reactor without causing a capacity overcharge in the same as well as in the distillation units of the urea recovery section.

Moreover, the additional synthesis reactor - to which substantially all the unreacted substances are recycled - can also operate at high conversion yield since only negligible amounts of water are fed to the same.

Because of this high conversion yield, it is advantageously possible to recover the urea produced in the second reactor directly in the pre-existing recovery section without causing a capacity overcharge of the distillation units.

## Claims

1. Process for urea production of the type comprising the steps of:
- performing a reaction between ammonia and carbon dioxide in a first reaction space (1) to obtain a reaction mixture comprising urea, carbamate and free ammonia in aqueous solution;
- feeding said mixture to a urea recovery section (3, 4, 6, 7);
- separating in said recovery section (3, 4, 6, 7) said carbamate and free ammonia in aqueous solution from the urea;
characterized in that it comprises the additional steps of:
- subjecting at least part of said carbamate and free ammonia in aqueous solution obtained in the urea recovery section (3, 4, 6, 7) to a treatment of partial decomposition of the carbamate and partial separation of the free ammonia (block 8) to obtain a flow comprising ammonia and carbon dioxide in vapor phase and a flow comprising residual carbamate in aqueous solution;
- subjecting said flow comprising ammonia and carbon dioxide in vapor phase to at least partial condensation (block 9) to obtain a flow of concentrate carbamate in aqueous solution;
- feeding at least part of said concentrate carbamate to a second reaction space (2);
- performing a reaction between ammonia and carbon dioxide in said second reaction space (2) to obtain a reaction mixture comprising urea, carbamate and free ammonia in aqueous solution.

2. Process according to claim 1, characterized in that the treatment of partial decomposition of the carbamate and partial separation of the free ammonia in aqueous solution (block 8) is carried out at a pressure substantially corresponding to the pressure in the second reaction space.

3. Process according to claim 1, characterized in that it further comprises the step of:
- feeding the flow comprising residual carbamate in aqueous solution resulting from the treatment of partial decomposition of the carbamate and partial separation of the free ammonia to said urea recovery section (3, 4, 6, 7).

4. Process according to claim 1, characterized in that the reaction mixture obtained in the second reaction space (2) is fed to said urea recovery section (3, 4, 6, 7).

5. Plant for urea production comprising:
- a first urea synthesis reactor (1);
- a second urea synthesis reactor (2);
- a recovery section (3, 4, 6, 7) of a reaction mixture comprising urea, carbamate and free ammonia in aqueous solution coming from said first urea synthesis reactor (1), for separating the urea from said carbamate and free ammonia in aqueous solution;
characterized in that it comprises:
- a stripping unit (8) for subjecting at least part of said carbamate and free ammonia in aqueous solution obtained in the urea recovery section (3, 4, 6, 7) to a treatment of partial decomposition of the carbamate and partial separation of the free ammonia to obtain a flow comprising ammonia and carbon dioxide in vapor phase and a flow comprising residual carbamate in aqueous solution;
- means (9) for condensing at least partially the vapors leaving said stripping unit (8) to obtain a flow of concentrate carbamate in aqueous solution;
- means (30) for feeding at least part of said concentrate carbamate to the second urea synthesis reactor (2).

6. Plant according to claim 5, characterized in that it further comprises:
- means (31) for feeding said flow comprising residual carbamate in aqueous solution from said stripping unit (8) to said recovery section (3, 4, 6, 7).

7. Plant according to claim 5, characterized in that it further comprises:
- means (24) for feeding a reaction mixture comprising urea, carbamate and free ammonia in aqueous solution leaving said second synthesis reactor (2) to said urea recovery section (3, 4, 6, 7).

8. Method for modernizing a plant for urea production of the type comprising:
- a first urea synthesis reactor (1);
- a recovery section (3, 4, 6, 7) of a reaction mixture comprising urea, carbamate and free ammonia in aqueous solution coming from said first urea synthesis reactor (1), for separating the urea from said carbamate and free ammonia in aqueous solution;
the method comprising the step of providing a second urea synthesis reactor (2);
characterized in that it further comprises the steps of:
- providing a stripping unit (8) for subjecting at least part of said carbamate and free ammonia in aqueous solution obtained in the urea recovery section (3, 4, 6, 7) to a treatment of partial decomposition of the carbamate and partial separation of the free ammonia to obtain a flow comprising ammonia and carbon dioxide in vapor phase and a flow comprising residual carbamate in aqueous solution;
- providing means (9) for condensing at least partially the vapors leaving said stripping unit (8) to obtain a flow of concentrate carbamate in aqueous solution;
- providing means (30) for feeding at least part of said concentrate carbamate to the second urea synthesis reactor (2).

9. Method according to claim 8, characterized in that it further comprises the step of:
- providing means (31) for feeding said flow comprising residual carbamate in aqueous solution from said stripping unit (8) to said recovery section (3, 4, 6, 7).

10. Method according to claim 8, characterized in that it further comprises the step of:
- providing means (24) for feeding a reaction mixture comprising urea, carbamate and free ammonia in aqueous solution leaving said second synthesis reactor (2) to said urea recovery section (3, 4, 6, 7).

## Patentansprüche

1. Verfahren zur Harnstoffherstellung, vom Typ her umfassend als Schritte:
- Durchführen einer Umsetzung zwischen Ammoniak und Kohlendioxid in einem ersten Reaktionsraum (1), wobei ein Reaktionsgemisch, bestehend aus Harnstoff, Carbamat und freiem Ammoniak in wässeriger Lösung, erhalten wird;
- Einspeisen des Gemisches in eine Harnstoff-Gewinnungszone (3, 4, 6, 7);
- Abtrennen des Carbamates und freien Ammoniaks in wässeriger Lösung vom Harnstoff im Harnstoffaufbereitungsabschnitt (3, 4, 6, 7);
dadurch gekennzeichnet, dass es als zusätzliche Schritte umfasst:
- zumindest einen Teil des in der Harnstoff-Gewinnungszone (3, 4, 6, 7) erhaltenen Carbamats und freien Ammoniaks in wässeriger Lösung einer Behandlung durch partielle Zersetzung des Carbamates und partielle Abtrennung des freien Ammoniaks (Block 8) zu unterziehen, um einen Stoffstrom, bestehend aus Ammoniak und Kohlendioxid in der Gasphase, und einen Stoffstrom, bestehend aus restlichem Carbamat in wässeriger Lösung, zu erhalten;
- den Stoffstrom, bestehend aus Ammoniak und Kohlendioxid in der Gasphase, einer zumindest partiellen Kondensation zu unterwerfen (Block 9), um einen Stoffstrom aus konzentriertem Carbamat in wässeriger Lösung zu erhalten;
- zumindest einen Teil des konzentrierten Carbamates in einen zweiten Reaktionsraum (2) einzuspeisen;
- eine Umsetzung zwischen Ammoniak und Kohlendioxid in dem zweiten Reaktionsraum (2) durchzuführen, um ein Reaktionsgemisch, bestehend aus Harnstoff, Carbamat und freiem Ammoniak in wässeriger Lösung, zu erhalten.

2. Verfahren nach Anspruch 1, dadurch gekennzeichnet, dass die Behandlung durch partielle Zersetzung des Carbamates und partielle Abtrennung des freien Ammoniaks in wässeriger Lösung (Block 8) bei einem Druck vorgenommen wird, der im wesentlichen dem Druck im zweiten Reaktionsraum entspricht.

3. Verfahren nach Anspruch 1, dadurch gekennzeichnet, dass es ferner als Schritt umfasst:
- Einspeisen des aus restlichem Carbamat in wässeriger Lösung bestehenden Stoffstromes, der aus der Behandlung durch partielle Zersetzung des Cabamats und partielle Abtrennung des freien Ammoniaks resultiert, in die Harnstoff-Gewinnungszone (3, 4, 6, 7).

4. Verfahren nach Anspruch 1, dadurch gekennzeichnet, dass das im zweiten Reaktionsraum (2) erhaltene Reaktionsgemisch in die Harnstoff-Gewinnungszone (3, 4, 6, 7) eingespeist wird.

5. Anlage zur Harnstoffherstellung, umfassend:
- einen ersten Harnstoffsynthesereaktor (1);
- einen zweiten Harnstoffsynthesereaktor (2);
- eine Gewinnungszone (3, 4, 6, 7) für ein Reaktionsgemisch, bestehend aus Harnstoff, Carbamat und freiem Ammoniak in wässeriger Lösung, welches aus dem ersten Harnstoffsynthesereaktor (1) stammt, zur Abtrennung des Harnstoffes von dem Carbamat und freien Ammoniak in wässeriger Lösung;
dadurch gekennzeichnet, dass sie umfasst:
- eine Stripper-Einheit (8), um zumindest einen Teil des in der Harnstoff-Gewinnungszone (3, 4, 6, 7) erhaltenen Carbamates und freien Ammoniaks in wässeriger Lösung einer Behandlung durch partielle Zersetzung des Carbamates und partielles Abtrennen des freien Ammoniaks zu unterziehen, wobei ein Stoffstrom, bestehend aus Ammoniak und Kohlendioxid in der Gasphase, und ein Stoffstrom, bestehend aus restlichem Carbamat in wässeriger Lösung, erhalten wird;
- Vorrichtung (9) für das zumindest partielle Kondensieren der Dämpfe, die die Stripper-Einheit (8) verlassen, um einen Stoffstrom aus konzentriertem Carbamat in wässeriger Lösung zu erhalten;
- Vorrichtung (30) für die Einspeisung von zumindest einem Teil des konzentrierten Carbamates in den zweiten Harnstoffsynthesereaktor (2).

6. Anlage nach Anspruch 5, dadurch gekennzeichnet, dass sie ferner umfasst:
- Vorrichtung (31) zur Einspeisung des aus restlichem Carbamat in wässeriger Lösung bestehenden Stoffstromes aus der Stripper-Einheit (8) in die Gewinnungszone (3, 4, 6, 7).

7. Anlage nach Anspruch 5, dadurch gekennzeichnet, dass sie ferner umfasst:
- Vorrichtung (24) zur Einspeisung eines aus Harnstoff, Carbamat und freiem Ammoniak in wässeriger Lösung bestehenden Reaktionsgemisches, das den zweiten Synthesereaktor (2) verlässt, in die Harnstoff-Gewinnungszone (3, 4, 6, 7).

8. Verfahren zur Modernisierung einer Anlage für die Harnstoffherstellung, welche vom Typ her umfasst:
- einen ersten Harnstoffsynthesereaktor (1);
- eine Gewinnungszone (3, 4, 6, 7) für ein aus Harnstoff, Carbamat und freiem Ammoniak in wässeriger Lösung bestehendes Reaktionsgemisch, das aus dem ersten Harnstoffsynthesereaktor (1) kommt, um den Harnstoff von dem Carbamat und freien Ammoniak in wässeriger Lösung zu trennen;
wobei das Verfahren den Schritt der Bereitstellung eines zweiten Harnstoffsynthesereaktors (2) umfasst;
dadurch gekennzeichnet, dass es ferner als Schritte umfasst:
- Bereitstellen einer Stripper-Einheit (8), um zumindest einen Teil des in der Harnstoff-Gewinnungszone (3, 4, 6, 7) erhaltenen Carbamates und freien Ammoniaks in wässeriger Lösung einer Behandlung durch partielle Zersetzung des Carbamates und partielle Abtrennung des freien Ammoniaks zu unterziehen, wobei ein Stoffstrom, bestehend aus Ammoniak und Kohlendioxid in der Gasphase, und ein Stoffstrom, bestehend aus restlichem Carbamat in wässeriger Lösung, erhalten wird;
- Bereitstellen von Vorrichtung (9) für das zumindest partielle Kondensieren der Dämpfe, die die Stripper-Einheit (8) verlassen, um einen Stoffstrom aus konzentriertem Carbamat in wässeriger Lösung zu erhalten;
- Bereitstellen von Vorrichtung (30) für die Einspeisung von zumindest einem Teil des konzentrierten Carbamates in den zweiten Harnstoffsynthesereaktor (2).

9. Verfahren nach Anspruch 8, dadurch gekennzeichnet, dass es ferner als Schritt umfasst:
- Bereitstellen von Vorrichtung (31) zur Einspeisung des aus restlichem Carbamat in wässeriger Lösung bestehenden Stoffstromes aus der Stripper-Einheit (8) in die Gewinnungszone (3, 4, 6, 7).

10. Verfahren nach Anspruch 8, dadurch gekennzeichnet, dass es ferner als Schritt umfasst:
- Bereitstellen von Vorrichtung (24) zur Einspeisung eines aus Harnstoff, Carbamat und freiem Ammoniak in wässeriger Lösung bestehenden Reaktionsgemisches, das den zweiten Synthesereaktor (2) verlässt, in die Gewinnungszone (3, 4, 6, 7).

## Revendications

1. Procédé de production d'urée du type comprenant les étapes consistant à :
- exécuter une réaction entre de l'ammoniac et du dioxyde de carbone dans un premier espace de réaction (1) pour obtenir un mélange réactionnel comprenant de l'urée, du carbamate et de l'ammoniac libre en solution aqueuse,
- envoyer ledit mélange à une section de récupération d'urée (3, 4, 6, 7),
- séparer de l'urée, dans ladite section de récupération (3, 4, 6, 7), lesdits carbamate et ammoniac libre,
caractérisé par le fait qu'il comprend les étapes supplémentaires consistant à :
- soumettre au moins une partie desdits carbamate et ammoniac libre en solution aqueuse, obtenus dans la section de récupération d'urée (3, 4, 6, 7), à un traitement de décomposition partielle du carbamate et séparation partielle de l'ammoniac libre (pavé 8) pour obtenir un courant comprenant de l'ammoniac et du dioxyde de carbone en phase vapeur et un courant comprenant du carbamate résiduel en solution aqueuse,
- soumettre ledit courant comprenant de l'ammoniac et du dioxyde de carbone en phase vapeur à condensation au moins partielle (pavé 9) pour obtenir un courant de carbamate concentré en solution aqueuse,
- envoyer au moins une partie dudit carbamate concentré à un deuxième espace de réaction (2),
- exécuter une réaction entre de l'ammoniac et du dioxyde de carbone dans ledit deuxième espace de réaction (2) pour obtenir un mélange réactionnel comprenant de l'urée, du carbamate et de l'ammoniac libre en solution aqueuse.

2. Procédé selon la revendication 1, caractérisé par le fait que le traitement de décomposition partielle du carbamate et séparation partielle de l'ammoniac libre en solution aqueuse (pavé 8) est effectué à une pression correspondant sensiblement à la pression dans le deuxième espace de réaction.

3. Procédé selon la revendication 1, caractérisé par le fait qu'il comprend en outre l'étape consistant à :
- envoyer à la section de récupération d'urée (3, 4, 6, 7) le courant comprenant du carbamate résiduel en solution aqueuse résultant du traitement de décomposition partielle du carbamate et séparation partielle de l'ammoniac libre.

4. Procédé selon la revendication 1, caractérisé par le fait que le mélange réactionnel obtenu dans le deuxième espace de réaction (2) est envoyé à la section de récupération d'urée (3, 4, 6, 7).

5. Installation de production d'urée comprenant :
- un premier réacteur de synthèse d'urée (1),
- un deuxième réacteur de synthèse d'urée (2),
- une section de récupération (3, 4, 6, 7) d'un mélange réactionnel comprenant de l'urée, du carbamate et de l'ammoniac libre en solution aqueuse venant dudit premier réacteur de synthèse d'urée (1), pour séparer l'urée desdits carbamate et ammoniac libre en solution aqueuse,
caractérisé par le fait qu'il comprend :
- un dispositif de dépouillement (8) pour soumettre au moins une partie desdits carbamate et ammoniac libre en solution aqueuse obtenus dans la section de récupération d'urée (3, 4, 6, 7) à un traitement de décomposition partielle du carbamate et séparation partielle de l'ammoniac libre pour obtenir un courant comprenant de l'ammoniac et du dioxyde de carbone en phase vapeur et un courant comprenant du carbamate résiduel en solution aqueuse,
- un moyen (9) pour condenser au moins partiellement les vapeurs qui quittent ledit dispositif de dépouillement (8) pour obtenir un courant de carbamate concentré en solution aqueuse,
- un moyen (30) pour envoyer au moins une partie dudit carbamate concentré au deuxième réacteur de synthèse d'urée (2).

6. Installation selon la revendication 5, caractérisée par le fait qu'elle comprend en outre :
- un moyen (31) pour envoyer, du dispositif de dépouillement (8) à la section de récupération (3, 4, 6, 7), le courant comprenant du carbamate résiduel en solution aqueuse.

7. Installation selon la revendication 5, caractérisée par le fait qu'elle comprend en outre :
- un moyen (24) pour envoyer à la section de récupération d'urée (3, 4, 6, 7) un mélange réactionnel comprenant de l'urée, du carbamate et l'ammoniac libre en solution aqueuse qui quitte le deuxième réacteur de synthèse (2).

8. Procédé de modernisation d'une installation de production d'urée du type comprenant :
- un premier réacteur de synthèse d'urée (1),
- une section de récupération (3, 4, 6, 7) d'un mélange réactionnel comprenant de l'urée, du carbamate et de l'ammoniac libre en solution aqueuse venant dudit premier réacteur de synthèse d'urée (1), pour séparer l'urée desdits carbamate et ammoniac libre en solution aqueuse,
ce procédé comprenant l'étape consistant à prévoir un deuxième réacteur de synthèse d'urée (2),
caractérisé par le fait qu'il comprend en outre les étapes consistant à :
- prévoir un dispositif de dépouillement (8) pour soumettre au moins une partie desdits carbamate et ammoniac libre en solution aqueuse, obtenus dans la section de récupération d'urée (3, 4, 6, 7), à un traitement de décomposition partielle du carbamate et séparation partielle de l'ammoniac libre pour obtenir un courant comprenant de l'ammoniac et du dioxyde de carbone en phase vapeur et un courant comprenant du carbamate résiduel en solution aqueuse,
- prévoir un moyen (9) pour condenser au moins partiellement les vapeurs qui quittent le dispositif de dépouillement (8) pour obtenir un courant de carbamate concentré en solution aqueuse,
- prévoir un moyen (30) pour envoyer au moins une partie dudit carbamate concentré au deuxième réacteur de synthèse d'urée (2).

9. Procédé selon la revendication 8, caractérisé par le fait qu'il comprend en outre l'étape consistant à :
- prévoir un moyen (31) pour envoyer du dispositif de dépouillement (8) à la section de récupération (3, 4, 6, 7) le courant comprenant du carbamate résiduel en solution aqueuse.

10. Procédé selon la revendication 8, caractérisé par le fait qu'il comprend en outre l'étape consistant à :
- prévoir un moyen (24) pour envoyer à la section de récupération d'urée (3, 4, 6, 7) un mélange réactionnel comprenant de l'urée, du carbamate et de l'ammoniac libre en solution qui quitte le deuxième réacteur de synthèse (2).
